# EUROPEAN PATENT APPLICATION

(11) **EP 3 888 530 A1**
(43) Date of publication of application: **06.10.2021**
(21) Application number: 20166717.7
(22) Date of filing: 30.03.2020
(51) Int. Cl.: A61B 5/00, A61B 5/1455, B63C 11/12, A63B 71/00

(54) **FACIAL DEVICE FOR MONITORING BIOMEDICAL PARAMETERS OF A USER**

(71) Applicant: Oxama S.r.l., 20145 Milano (IT)
(72) Inventor: Mattavelli, Claudio, 20865 Usmate Velate Monza Brianza (IT); Moi, Massimo, 20871 Vimercate Monza Brianza (IT); Palumbo, Vincenzo, 20131 Milano (IT)
(74) Representative: Siniscalco, Fabio

(57) **Abstract**

A face device (49) for monitoring biomedical parameters of a user provides a support and housing structure shaped so as to be applied to the head (1) of the user, an oximetry sensor (53) fitted to the support and housing structure so as to adhere, in an operating position, to the face of the user at the facial artery, a sound source (54) fitted to the support and housing structure so as to adhere, in an operating position, to a cranial bone of the user, an independently powered electronic board (55) for processing data, received in the support and housing structure and connected to the oximetry sensor (53) and to the sound source (54) so as to detect and process signals coming from the oximetry sensor (53) and to provide information on biomedical parameters to the user acoustically through the sound source (54). This face device (49) does not engage or distract the user.

## Description

### BACKGROUND OF THE INVENTION

This invention relates to a face device that is able to monitor biomedical parameters of a user wearing the face device.

### PRIOR ART

In many professions in a hostile environment, for example with a high chemical or biological risk, or in environments potentially lacking in oxygen, the operators need to protect their faces with personal protective equipment. For example, in the case of protective equipment for the respiratory tract, operators use masks with or without filters, half-masks or full face masks, aqualungs or respirators with external air supply, or in the case of protective equipment for the eyes, the operators use goggles, masks, visors and screens. In most of the aforesaid professions, it would be desirable to monitor heartbeat and oxygen saturation in the blood of the operator to ensure that environmental conditions have not compromised breathing capacity and thus the health of the operator or have not caused the operator to lose consciousness.

Many sporting activities, both those considered to be extreme such as for example scuba diving in apnoea or with aqualungs, or parachuting, paragliding, wingsuit flying, or mountaineering, free climbing, extreme downhill cycling, but also sporting activities that are considered to be non-extreme disciplines like cycling, skiing and snowboarding involve an intense psychomotor and breathing effort accompanied by a high level of adrenaline and also involve the hands being engaged by the activity. Also in these cases it is important, both during the training phase and also during the sporting discipline proper, to be able to measure the biomedical parameters like heartbeat and oxygen saturation in the blood.

For example, in scuba diving in apnoea, during the apnoea phase, the level of oxygen in the blood decreases continuously and on the other hand the level of carbon dioxide increases, at a speed that depends on various factors such as for example the heartbeat frequency, the temperature of the water, muscular effort, psychophysical conditions, and other things. The human organism is not able to evaluate the quantity of oxygen in the blood, but manages to send some signals, such as for example contractions of the diaphragm, which are triggered exclusively by an increase in carbon dioxide. Nevertheless, these signals are not easily interpretable and also an expert freediver could be mistaken in evaluating the availability of oxygen during the immersion.

Should the level of oxygen fall below a certain threshold, the freediver would experience a temporary loss of psychomotor control accompanied by a sensation of weakness, trembling, blurred vision and poor motor coordination. If in this phase the freediver did not start breathing again, for example following a prompt assistance, the freediver could suffer a serious syncope event, also called "black-out", which causes the loss of consciousness and total inability to act. However, the human body reacts to a syncope with an extraordinarily fortunate response, i.e. by a laryngospasm that, by contacting the glottis, occludes the throat. This involuntary mechanism prevents the water entering the lungs and in fact prevents drowning. In this situation, the freediver can still be saved, for a very small number of minutes, before the lack of oxygen causes the main organs of the organisms to deteriorate with consequent and inevitable death.

It is thus clear how desirable it is to be able to measure the oxygen level in the blood of the freediver, both during the training phase in order to gain knowledge of the physical condition of the freediver and in order to improve continuously sporting performance, and during deep immersion in a natural environment in order to increase the safety of the freediver.

Devices are known that are suitable for measuring the level of oxygen saturation in the blood, which are also called oximeters or saturation meters or pulse oximeters when they also measure heartbeat.

As the most common commercial oximeter probes are applied to the index finger of the hand, it is clear that use thereof is greatly limited in all the human activities listed previously for which the hands are engaged in the activity.

In particular, during sleep, an oximeter probe positioned on the index finger will be subject to continuous and involuntary movements of the hand that would lead to errors in the reading of the plethysmographic signal. During the activities of operators in an environment that is hostile or lacking in oxygen such as firefighters and maintenance workers of cisterns, silos, conduits, mines, or operators exposed to a contaminated environment with a high chemical or biological risk, or operators operating at altitude or underwater, it is clear that an oximetry probe positioned on the index finger of the hand would make it difficult to perform any manual task, compromising the objectives of the professional activity. Similarly, during the sports activities disclosed above, as the hands are engaged in the sporting activity, the use of an oximetry probe positioned on the index finger would compromise correct performance of the sporting activity.

A particular mention must be made of aerodynamic sports such as for example parachuting and wingsuit flying, and of hydrodynamic sports such as for example swimming or scuba diving in apnoea. For such sports the hands and arms are used to maintain the aerodynamic and hydrodynamic position, so it is not possible to position any probe or instrument on a finger, on the wrist or on the arm because this would compromise the movement thereof, or the movement of the limbs would compromise correct measurement of the biomedical parameters. Further, any device provided with a display on the wrist would be impossible to read or display because such an operation would require the arm to be moved from the optimum position necessary for the aerodynamic or hydrodynamic setup.

### OBJECTS OF THE INVENTION

One object of the present invention is to provide a face device for monitoring biomedical parameters of a user that enables the disclosed limits to be overcome.

A further object of the present invention is for the aforesaid face device to be able to communicate the biomedical data to the user without causing distractions to the user that may disturb the action of the user.

### BRIEF DESCRIPTION OF THE INVENTION

Such objects are achieved by a face device for monitoring biomedical parameters of a user in accordance with the first claim.

### BRIEF DESCRIPTION OF THE DRAWINGS

In order to better understand the invention, a description is given below of a series of non-limiting exemplary embodiments of the invention, which are illustrated in the attached drawings in which:
Figs 1, 2 show the operating principle of the face device for monitoring biomedical parameters of a user, in accordance with the invention;
Fig.3 is a block diagram that shows the structure and operation of the face device in accordance with the invention;
Figs 4, 5, 6 show in a perspective view a first embodiment of a face device according to the invention;
Figs 7, 8, 9 show in a perspective view, in a rear view, and in a lateral section respectively, a second embodiment of a face device according to the invention;
Fig. 10 shows two views, perspective and in a lateral section respectively, of a component of the face device of Figs 7-9;
Fig.11 shows two side views of the component of Fig.10, illustrating the operating dynamic of the component;
Figs 12, 13 show, in a perspective view, in a front view and in a side view, a third embodiment of a face device according to the invention;
Figs 14, 15 show a perspective view of the face device of Figs 12, 13 associated with a diving mask;
Figs 16, 17 show a perspective view of the face device of Figs 12, 13 associated with a gas mask;
Figs 18, 19 show, in a perspective view, in a front view and in a side view, a fourth embodiment of a face device according to the invention;
Figs 20, 21 show a perspective view of the face device of Figs 18, 19 associated with swimming goggles;
Figs 22, 23 show a perspective view of the face device of Figs 18, 19 associated with a ski mask.

### DETAILED DESCRIPTION OF THE INVENTION

The illustrated face devices are able to measure heartbeat and oxygen saturation in the blood.

In particular, with regard to oxygen saturation in the blood, these devices enable the concentration of haemoglobin to be measured that, in this specific case, is linked to the oxygen. It is known that the haemoglobin bound to oxygen has a light absorption spectrum that is rather different from that of haemoglobin that is not bound and by measuring the plethysmographic signal, i.e. measurements of light absorption during volume variations of the blood vessels between the systolic phase, in which the heart compresses the blood in the arteries, and the diastolic phase, during which the heart relaxes, it is possible to calculate oxygen saturation in the blood.

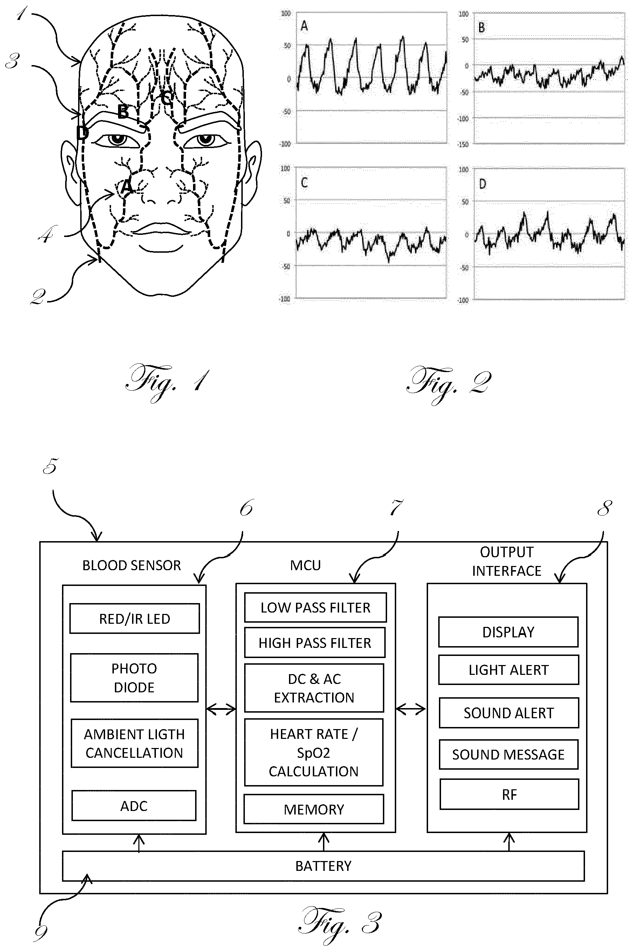

In Fig.1 the diagram is shown of the arterial system of the head 1 of a person, consisting of the carotid artery 2 that divides into the temporary artery 3 and into the facial artery 4, in a symmetrical and specular manner both to the right and left of the face. In particular, it is clear that the facial artery 4 traverses the cheek spreading horizontally in a region of the face between the cheekbone and the nostril and vertically in the direction of the eye.

In Fig.2 plethysmographic signals are shown that are measured with an oximetry probe placed on the head 1 at the facial artery (A), of the eyebrow (B), of the forehead (C) and of the temporal artery (D), in rest conditions, at ambient temperature, at atmospheric pressure, and with the same instrument and person. These measurements show that the signal relating to the facial artery (A) is the widest signal.

The illustrated face devices all have an oximetry probe arranged at the facial artery 4 to measure in an optimum manner the heartbeat and oxygen saturation in the blood. In Fig.3 the block diagram is shown that is common to all the illustrated face devices, which shows the structure and operation thereof. Each face device has a support and housing structure, indicated by 5. The structure 5 supports and contains an oximetry sensor 6, a processing unit 7, an interface assembly 8 and an energy storage unit 9. The oximetry sensor 6 has a plurality of light sources at different wavelengths, for example light emission diodes, and a plurality of light sensors, for example photodiodes, an ambient light suppression circuit and an analogue-digital converter (ADC). The digital signal is processed by the processing unit 7 (MCU), which comprises numeric low-pass and high-pass filters, or equivalently band-pass filters, that enable the direct-current (DC) and alternating-current (AC) component of the plethysmographic signal to be extracted. By measuring these values it is possible to calculate heartbeat frequency, and the number of repetitions of the AC signal in the time unit, and oxygen saturation in the blood. The aforesaid biomedical parameters, heartbeat frequency and oxygen saturation in the blood, are communicated by the interface system 8 as a source of sounds and a plurality of light sources that are visible both from the inside and the outside of the mask. The aforesaid electronic components are supplied by the energy storage unit 9 so as to make each face device independent during the dive.

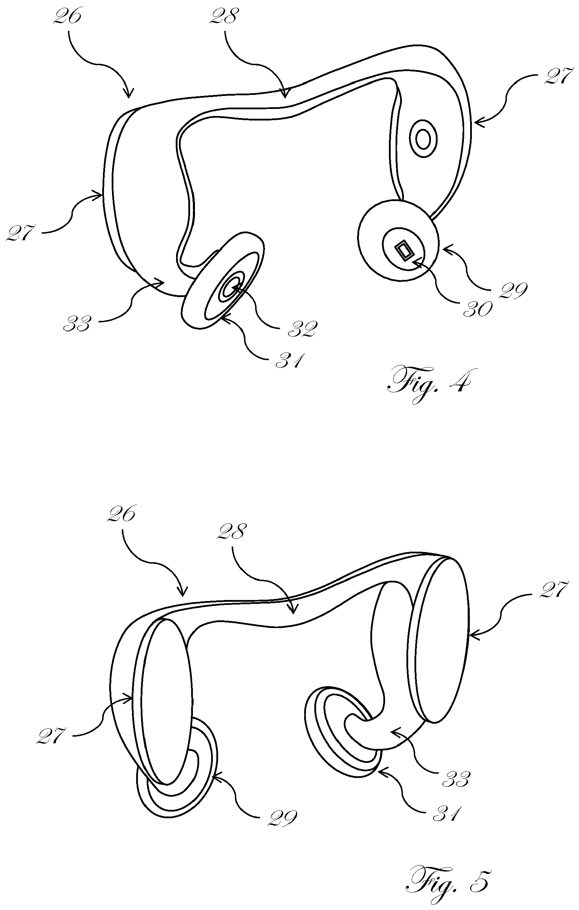

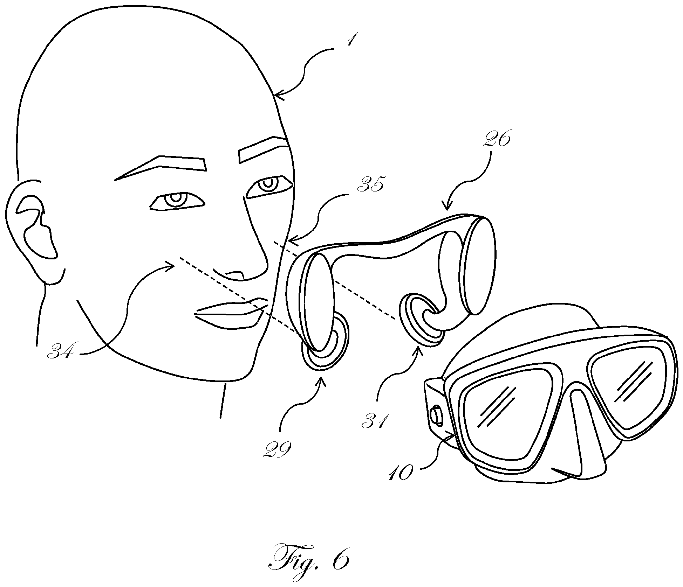

In Figs 4-6 a face device is illustrated that is intended for applications in the sport area, in association with a diving mask 10. The face device, indicated by 26, has a body provided with two elastic extensions 27 connected by a bridge 28, which is also elastic. The processing unit 7 is included in the body with the interfaces 8 and the energy storage unit 9. The elastic extensions 27, by expanding, press against the inner walls of the eye cavities of the diving mask 10, maintaining the device 26 integral with the mask. In Figs 4, 5 two views of the device 26 are shown, in which are highlighted a first disc-shaped casing 29, at the end of an elastic extension 27, in which an oximetry sensor 30 is included and a second disc-shaped casing 31, at the end of the other elastic extension 27, in which a sound source 32 is included, for example an electromechanical bone conduction transducer. The aforesaid casings 29 and 31 are connected to the body of the device 26 by two elastic arms 33 that enable the oximetry sensor 30 and the sound source 32 to be maintained pressed at constant pressure onto the face of the diver. As shown better in Fig.6, the device 26 is housed in the diving mask 10, in such a manner that the oximetry sensor 29 is positioned at the facial artery 34 of the head 1 of the diver at constant pressure, to avoid false signals of the plethysmographic reading, more precisely in a region located horizontally between one of the two cheekbones and the nose and vertically below the eye. Similarly, the sound source 31 positions itself at the other cheekbone, indicated by 35, of the head 1 of the diver at constant pressure, so that the acoustic signal can propagate through the bones of the cranium and be heard by the diver; in particular, the electromechanical transducer converts electric signals, corresponding to the sound to be transmitted, into mechanical vibrations to transmit the sound to the inner ear through the bones of the cranium.

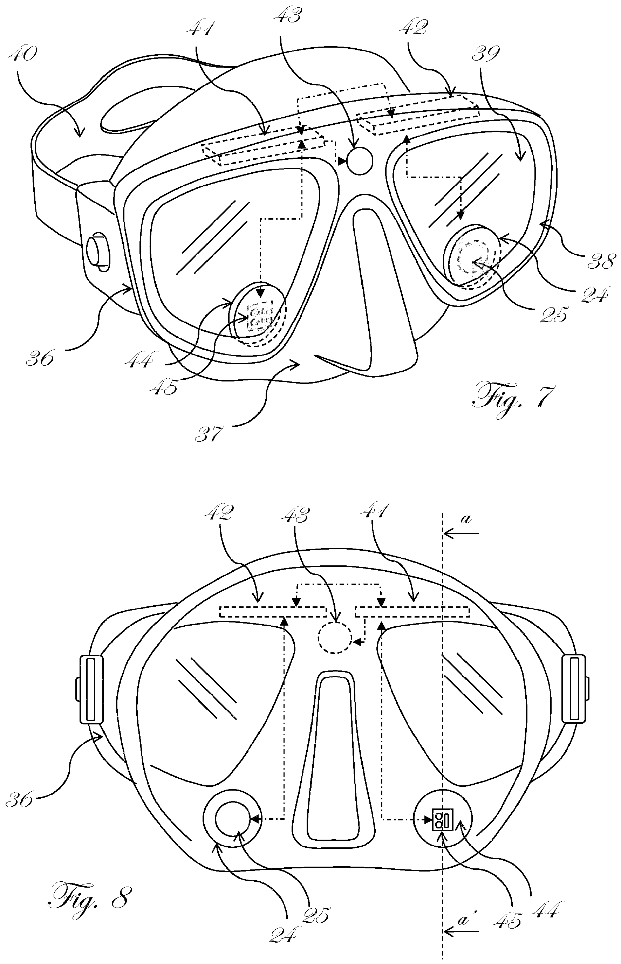

In Figs 7-9 a face device is illustrated that provides a protective face mask 36, for example a diving mask, having a soft structure 37 that adapts to the face of the diver, a stiff structure or frame 38 that supports one or two windows of transparent material 39, and one or more adjustable elastic straps 40 that secure the mask to the face of the diver. In this embodiment of the device, the soft structure 37 has, below one of the two windows 39, a flexible extension 44 that contains an oximetry sensor 45. Further, the soft structure 37 has, below the other of the two windows 39, a flexible extension 24, which is similar to the flexible extension 44, that contains a sound source 25, for example an electromechanical bone conduction transducer. The stiff structure 38 houses electronic boards 41 and 42, on which both the processing unit 7 and the energy storage unit 9 are present, connected electrically to one another and to the oximetry sensor 45 and the sound source 25. Also, the frame 38 contains one or more light sources 43 that are visible by other operators from the outside of the mask that can signal a risk situation for the health of the user.

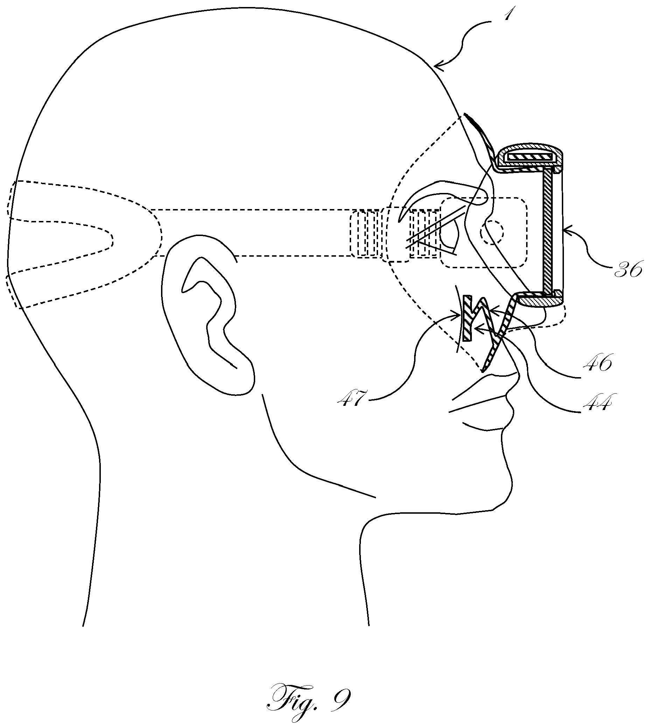

In Fig.9 the positioning of the mask 36 on the head 1 of a user is indicated, for example a diver, according to the section in plane bb' of Fig.8. In particular, it is shown how the flexible extension 44 has an elastic arm 46 that maintains the oximetry sensor pressed against the face of the user, at the facial artery 47, more precisely in a region located horizontally between the cheekbone and the nose and vertically below the eye, with an appropriate pressure. Excessive pressure would cause compression of the arterial vessels and, on the other hand, insufficient pressure would cause empty spaces to be created, in both cases causing false optical signals and thus measuring errors.

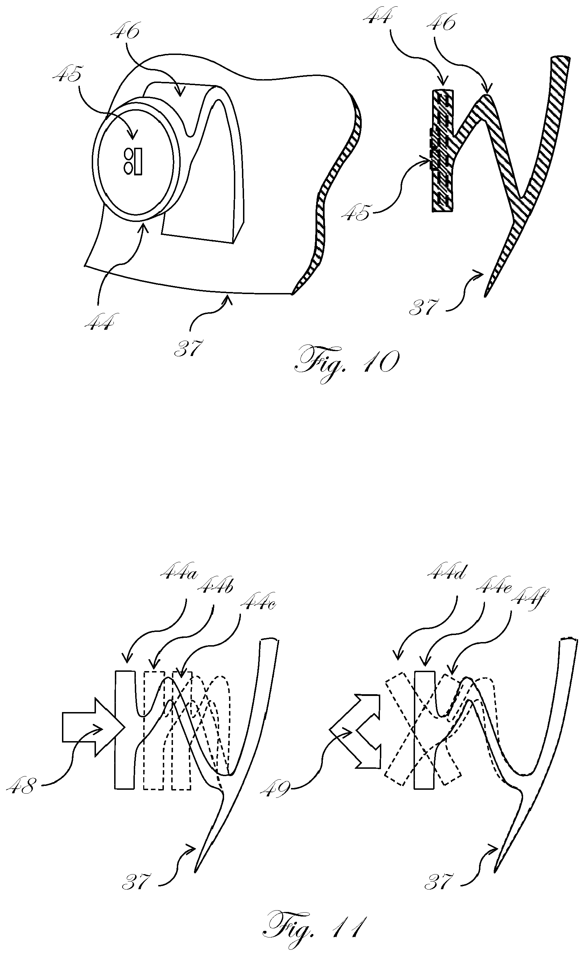

Fig. 10 shows the detail of the flexible extension 44 with the oximetry sensor 45, and in particular the connection of this flexible extension 44 with the edge of the soft structure 37 of the mask 36 through the elastic arm 46 is clearly visible.

In Fig.11 the operating dynamics are illustrated of the flexible extension 44 and of the elastic arm thereof; in particular the flexible extension at rest (44a and 44e, continuous line) is shown and on the other hand the flexible extension when it is subject to deformation caused by a push 48 (44b and 44c, dashed line) and a twist 49 (44d and 44f, dashed line) by the cheekbone of the user on the oximetry sensor.

During the dive, for example, because of the variation in water pressure but also because of the pressure variations due to the voluntary compensation of the diver, the mask 36 can vary the distance from the face of the diver. It is clear that owing to the shape and elasticity of the arm 46, the oximetry sensor 45 remains positioned at the facial artery of the diver at constant pressure, avoiding false signals of the plethysmographic reading.

Similar technical considerations can apply to the flexible extension 24 containing the sound source 25.

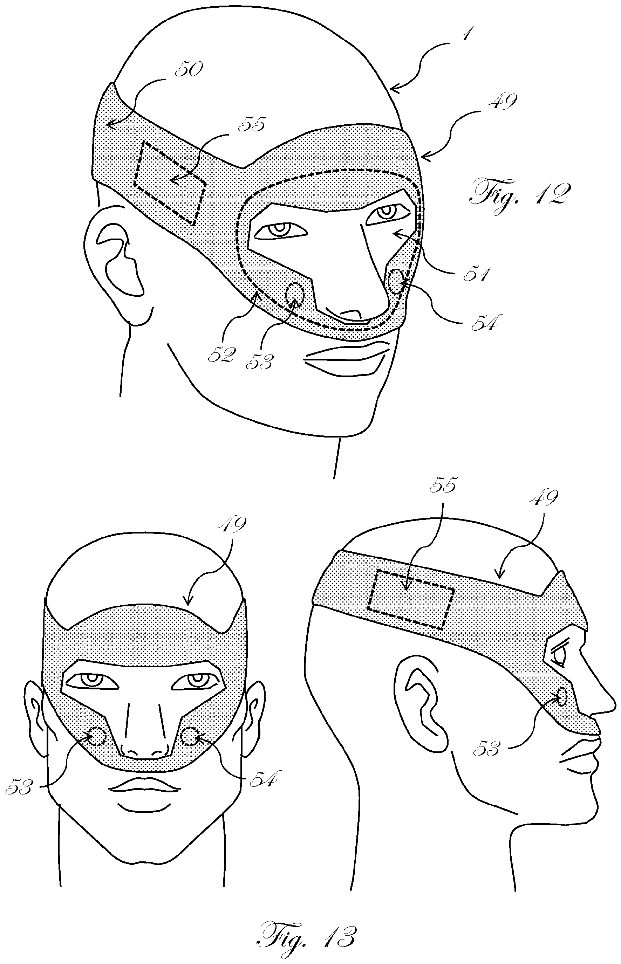

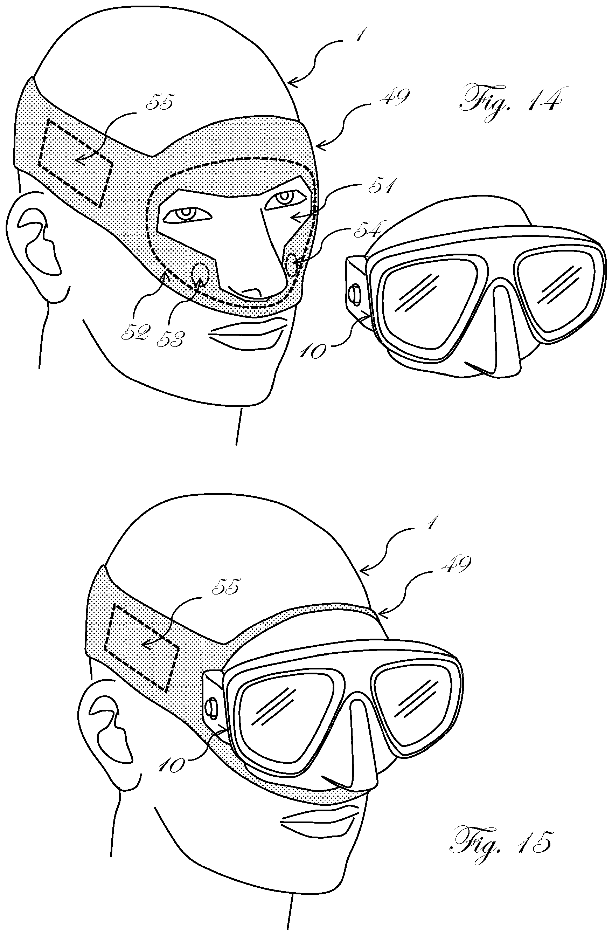

In Figs 12-17 the device has the configuration of a soft face mask 49, the latter having a casing made of elastic material, for example rubber, silicone, silicone rubber or any elastic polymer, or other, and of a moderate thickness (for example in the range between 1 and 3 mm), so as to be worn on the head 1 of a person and be supported by one or more appropriate straps 50 that are fastened behind the head 1. This face mask 49 leaves the eyes and the nose uncovered by an opening 51 of a continuous face portion 52 of the casing (dashed line) that extends around the eyes, on the forehead and below the nose. The mask 49 is combinable with the diving mask 10 viewed previously and the continuous portion 52 of the mask 49 does not have any discontinuity along the surface and is able to ensure a hermetic seal between the mask 10 and the face of the user, as shown in Figs 14,15. Inside the casing of the mask 49, included inside cavities of greater dimensions with respect to the rest of the face mask (for example in the range between 1 and 10 mm) an oximetry sensor 53 and a sound source 54 are housed, together with an electronic board 55, including the processing unit 7 and the energy storage unit 9.

In particular, the oximetry sensor 53 is positioned at the facial artery of the user at a constant pressure, avoiding false signals of the plethysmographic reading, more precisely in a region located horizontally between a cheekbone and the nose and vertically below the eye. Similarly, the sound source 54 is positioned at the other cheekbone of the user at a constant pressure, so that the acoustic signal can propagate through the bones of the cranium and be heard by the user. Both the oximetry sensor 53 and the sound source 54 are positioned inside or outside the continuous portion 52 so that the pressure thereof on the face is not affected by the involuntary movements of the mask.

In application use, for example for dives, Figs 14,15 show the face mask 49 worn by a user on his head 1, before and after wearing the diving mask 10. The latter adheres perfectly to the face of the user owing to the fact that between the diving mask 10 and the face there is the continuous portion 52 of the face mask 49, which does not have any discontinuity and ensures the hermetic seal from the surrounding environment.

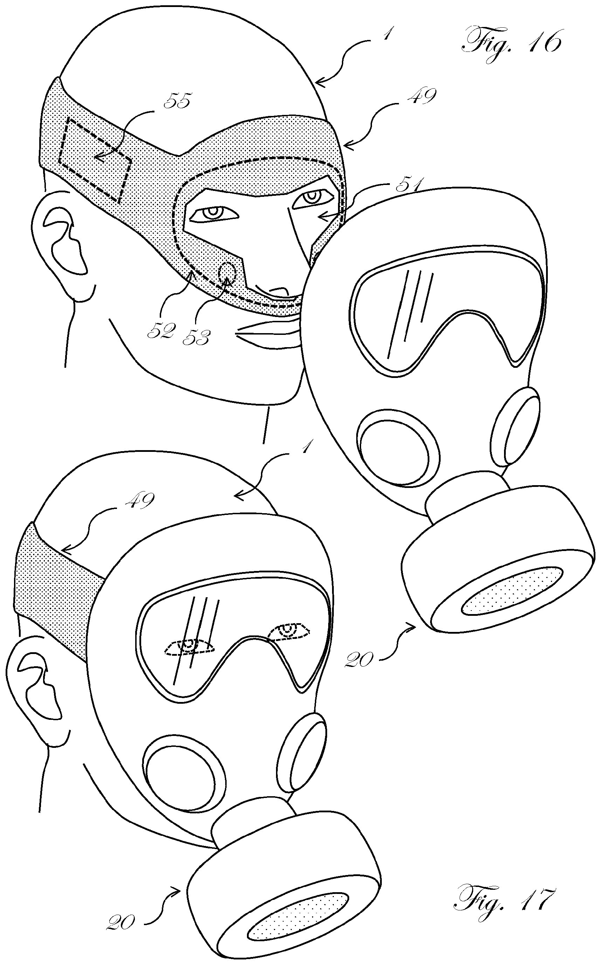

For any use in hostile environments, in Figs 16,17 the face mask 49 is shown for measuring the oxygen saturation in the blood worn by a user on his head 1, before and after wearing a protective face mask, which in this embodiment is a gas mask 20. The latter adheres to the face of the user owing to the fact that between the gas mask 20 and the face there is the continuous portion 52 of the face mask 49, which does not have any discontinuity and ensures the hermetic seal from the surrounding environment.

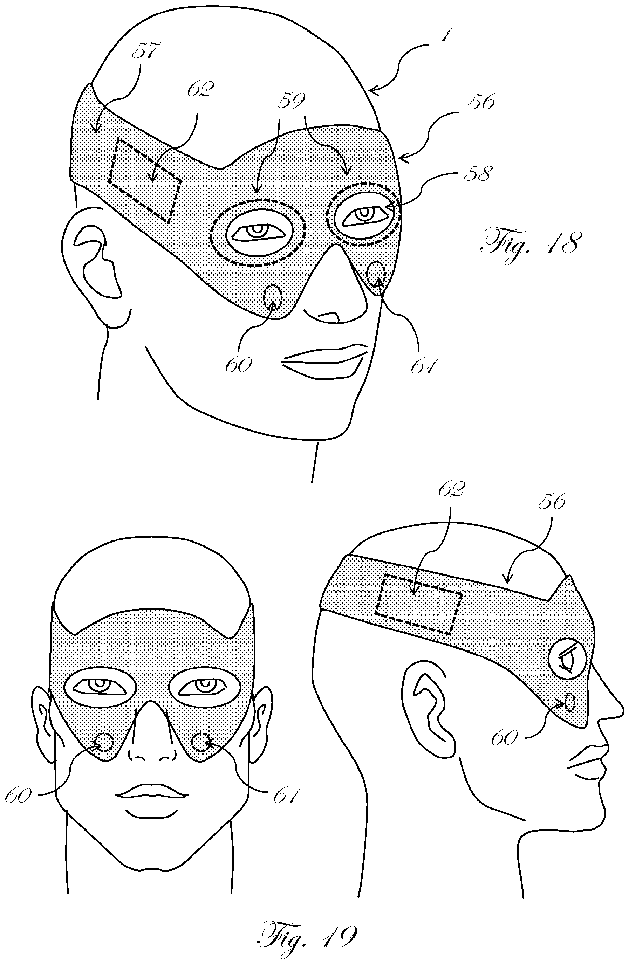

Figs 18-23 show a soft face mask 56 that is similar to the preceding mask 49.

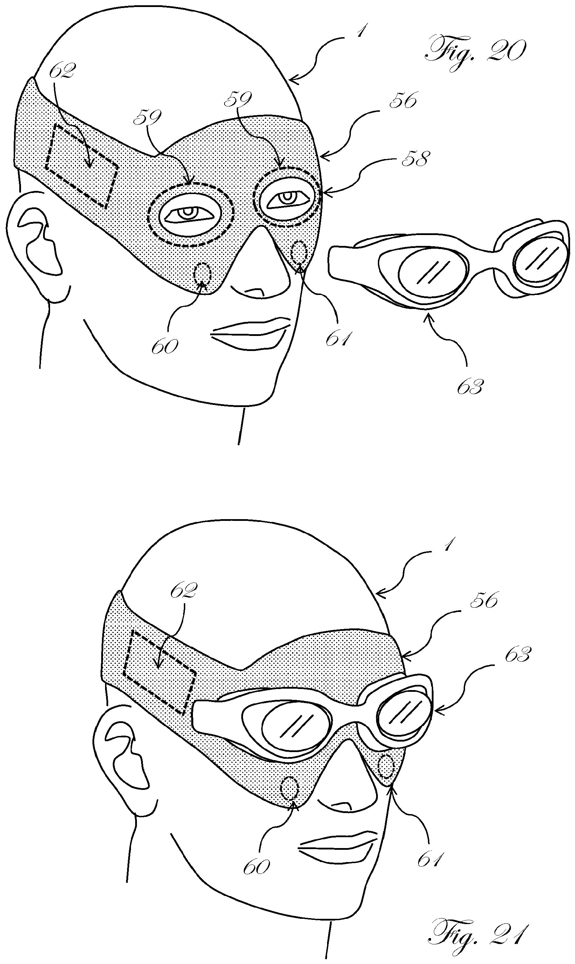

The mask 56 has a casing made of elastic material, for example rubber, silicone, silicone rubber or any elastic polymer, or other, and with a low thickness (for example in the range between 1 and 3 mm), so as to be worn on the head 1 of a person and supported by one or more suitable straps 57 that are tied behind the head 1. This face mask leaves the eyes uncovered by two openings 58 of two continuous portions 59 of the casing around the eyes (dashed lines). Such a mask 56 is combinable with swimming goggles 63 and its continuous portions 59 do not have discontinuity along the surfaces thereof and are able to ensure the hermetic seal between the goggles 63 and the face of the user, as shown in Figs 20, 21. Inside the casing, included inside cavities of greater dimensions with respect to the rest of the face mask (for example in the range between 1 and 10 mm), an oximetry sensor 60 and a sound source 61 are housed, together with an electronic board 62, including the processing unit 7 and the energy storage unit 9.

In particular, the oximetry sensor 60 is positioned at the facial artery of the user at a constant pressure avoiding false signals of the plethysmographic reading, more precisely in a region located horizontally between a cheekbone and the nose and vertically below the eye. Similarly, the sound source 61 is positioned at the other cheekbone of the user at constant pressure, so that the acoustic signal can propagate through the bones of the cranium and be heard by the user. Both the oximetry sensor 60 and the sound source 61 are positioned outside the continuous portions 58 so that the pressure thereof on the face is not affected by the involuntary movements of the mask or of the protective goggles.

Figs 20, 21 show the face mask 56 worn by a user on his head 1, before and after wearing the swimming goggles 63. The latter adhere perfectly to the face of the user owing to the fact that between the swimming goggles and the face the continuous portions 59 of the face mask 56 are located that do not have any discontinuity and ensure the hermetic seal from the surrounding environment.

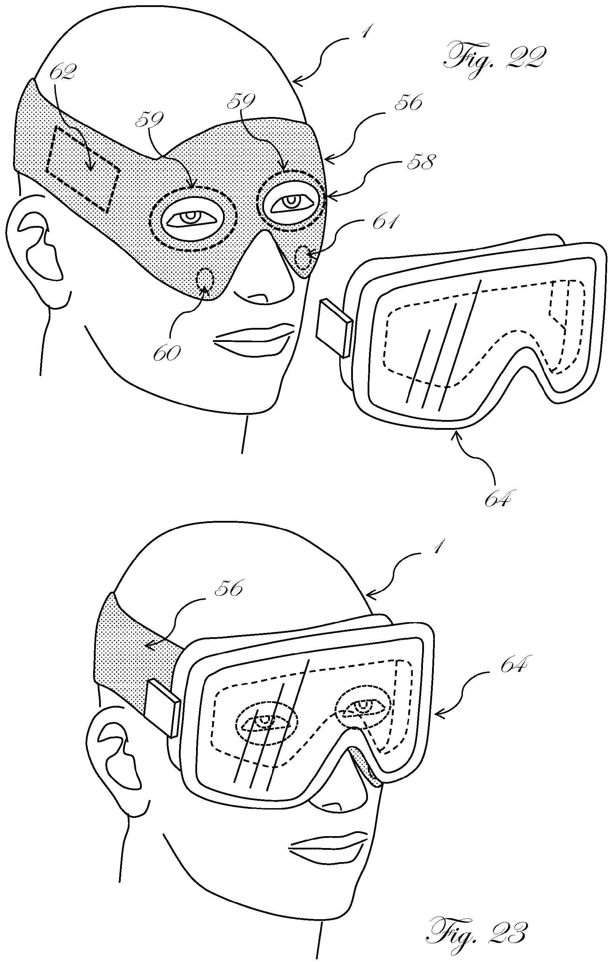

In application use in other sports such as for example parachuting, paragliding, wingsuit flying, mountaineering, freeclimbing, cycling, skiing, snowboarding, and the like, the mask 56 is combinable with a ski mask 64. In Figs 22, 23, the mask 56 is shown worn by a user on his head 1, before and after wearing the mask 64. This mask 64 is worn by the user above the face mask 56 owing to the fact that between the mask 64 and the face there are the continuous portions 59 of the face mask 56.

Both in the face mask 49 and in the face mask 56, the sound source can be an electromechanical bone conduction transducer.

The face masks 49 and 56 can also be used in the absence of any protective mask or goggles, in all the uses in which it is necessary to measure the biomedical parameters like heartbeat and oxygen saturation in the blood continuously and without hampering body movements, like for example:
- for oximetry measurements in intensive care both on health workers and patients, in particular in the case of epidemics;
- during sleep for monitoring sleep apnea;
- during professional activities that are critical for the health of the user;
- in floor gymnastic disciplines.

The disclosed and illustrated face devices permit monitoring of biomedical parameters of a user without engaging the user in any manner.

Further, such face devices are able to communicate biomedical data to the user acoustically without causing distractions to the user that may disturb the actions of the user.

Variations can be made to both the general configuration and the configuration of the components of the illustrated face devices.

Other types of sensor can be provided that are incorporated into the face device to measure biomedical parameters such as body temperature sensors, arterial pressure sensors or other sensors.

Sensors can further be provided for measuring operating parameters such as external temperature sensors, depth sensors, GPS, accelerometers or other sensors.

The face devices 26, 49, 56 can be combined with any type of protective mask or protective goggles for any application.

## Claims

1. Face device (26;36;49;56) for monitoring biomedical parameters of a user, comprising a support and housing structure shaped so as to be applied to the head (1) of the user, an oximetry sensor (30;45;53;60) fitted to the support and housing structure so as to adhere, in an operating position, to the face of the user at the facial artery, at least one sound source (25;32;54;61) fitted to the support and housing structure so as to adhere, in an operating position, to a cranial bone of the user, an independently supplied processing unit (7) received in the support and housing structure and connected to the oximetry sensor (30;45;53;60) and to the sound source (25;32;54;61) so as to detect and process signals coming from the oximetry sensor (30;45;53;60) and to provide information on biomedical parameters to the user acoustically through the sound source (25;32;54;61).

2. Face device according to claim 1, comprising a body provided with elastic extensions (27) connected by an elastic bridge (28), wherein the processing unit (7) is included in the body, wherein the oximetry sensor (30) is included in a respective first disc-shaped casing (29) and the sound source (32) is included in a respective second disc-shaped casing (31), wherein the casings (29,31) are connected to elastic extensions (27), and wherein the body is associated with a protective face mask (10).

3. Face device according to claim 2, wherein the casings (29, 31) are connected to elastic extensions (27) by two respective elastic arms (33) for maintaining the oximetry sensor (30) and the sound source (32) pressed at a constant pressure onto the face of the user.

4. Face device according to claim 1, comprising a protective face mask (36) for protecting the face of the user, having a soft structure (37) that adapts to the face and a stiff structure (38) that supports at least one window (39) of transparent material, and having one or more adjustable elastic straps (40) that secure the mask (36) on the face, wherein the soft structure (37) has, below the window (39) or the windows (39), a first flexible extension (44) containing the oximetry sensor (45) and a second flexible extension (24) containing the sound source (25), and wherein the processing unit (7) is included in the stiff structure (38).

5. Face device according to claim 4, wherein each flexible extension (24, 44) is connected to the soft structure (37) by an elastic arm (46).

6. Face device according to claim 1, comprising a soft face mask (49) having a casing made of elastic material, suitable for being worn on the head (1) of the user and supported by one or more straps (50) that are fastened behind the head (1), wherein the face mask (49) has an opening (51) of a continuous face portion (52) of the casing that, when the mask is worn, extends around the eyes, on the forehead and below the nose of the user, and wherein the oximetry sensor (53), the sound source (54) and the processing unit (7) are incorporated into the casing.

7. Face device according to claim 6, wherein the soft mask (49) is associated with a protective mask (10; 56).

8. Face device according to claim 1, comprising a soft face mask (56) having a casing made of elastic material, which is suitable for being worn on the head (1) of the user and is supported by one or more straps (57) that are tied behind the head (1), wherein the face mask (56) has two openings (58) of two respective continuous face portions (59) of the casing that, when the mask is worn, extend around the eyes, and in which the oximetry sensor (60), the sound source (61) and the processing unit (7) are incorporated into the casing.

9. Face device according to claim 8, wherein the soft face mask (56) is associated with protective goggles (63).

10. Face device according to claim 8, wherein the soft face mask (56) is associated with a protective mask (64).

11. Face device according to any one of the preceding claims, wherein the sound source (25; 32; 54; 61) is an electromechanical bone conduction transducer.

12. Device according to any one of the preceding claims, incorporating operating parameter sensors connected to the processing unit (7).

13. Device according to any one of the preceding claims, incorporating one or more light sources (43) connected to the processing unit (7), visible by the user and/or from the exterior, to signal danger situations.
